# EUROPÄISCHE PATENTANMELDUNG

(11) **EP 3 748 620 A1**
(43) Veröffentlichungstag der Anmeldung: **09.12.2020**
(21) Anmeldenummer: 20176447.9
(22) Anmeldetag: 26.05.2020
(51) Int. Cl.: G09G 3/20, G16H 40/20

(54) **ANZEIGESYSTEM UND VERFAHREN ZUM ANZEIGEN EINER AUSGABE EINES ELEKTROMEDIZINISCHEN GERÄTES**

(30) Priorität: 07.06.2019 DE 102019003995
(71) Anmelder: Drägerwerk AG & Co. KGaA, 23558 Lübeck (DE)
(72) Erfinder: Schlichting, Stefan, 23558 Lübeck (DE); Frost, Jan, 23558 Lübeck (DE); Merkel, Maximilian, 23558 Lübeck (DE); Brandt, Christian, 23558 Lübeck (DE); Scheuplein, Sina, 23558 Lübeck (DE)

(57) **Zusammenfassung**

Die Erfindung betrifft ein Anzeigesystem (100) zum Anzeigen einer Ausgabe eines elektromedizinischen Gerätes (105), mit einer Anzeigeeinheit (110), einer Anzeigesignaleinheit (120) und einer Anzeigesignalüberwachungseinheit (130). Die Anzeigeeinheit ist ausgebildet, ein Anzeigesignal (112) zu empfangen und basierend auf dem Anzeigesignal eine visuelle Ausgabe (114) darzustellen. Die Anzeigesignaleinheit ist ausgebildet, Daten (107), die das elektromedizinische Gerät betreffen, zu empfangen, diese Daten einem Visualisierungstyp zuzuordnen, abhängig von den aktuell darzustellenden Visualisierungstypen eine jeweilige Darstellungsart der empfangenen Daten dynamisch zu bestimmen und basierend auf den Daten und der jeweils bestimmten Darstellungsart das Anzeigesignal zu erzeugen und an die Anzeigeeinheit auszugeben. Die Anzeigesignalüberwachungseinheit ist mit der Anzeigeeinheit und der Anzeigesignaleinheit signaltechnisch verbunden und ist ausgebildet, das Anzeigesignal zu empfangen und ein Ausgabesignal (118) von der Anzeigeeinheit zu empfangen, wobei das Ausgabesignal Informationen zur erfolgten visuelle Ausgabe enthält. Dabei ist die Anzeigesignalüberwachungseinheit ausgebildet, Anzeigesignal und Ausgabesignal miteinander zu vergleichen und im Falle einer mangelnden Übereinstimmung zwischen erfolgter visueller Ausgabe und zu erfolgender visueller Ausgabe ein Warnsignal (134) auszugeben.

## Beschreibung

Die Erfindung betrifft ein Anzeigesystem zum Anzeigen einer Ausgabe mindestens eines elektromedizinischen Gerätes und ein entsprechendes Verfahren zum Anzeigen einer Ausgabe mindestens eines elektromedizinischen Gerätes. Weiterhin betrifft die Erfindung ein Programm mit einem Programmcode zur Durchführung des erfindungsgemäßen Verfahrens.

Im medizinischen Umfeld ist die Verwendung einer Anzeigeeinheit bekannt, die die Daten eines angeschlossenen elektromedizinischen Gerätes anzeigt und mit dem Betriebsmodus dieses elektromedizinischen Gerätes synchronisiert ist.

US 9,104,789 B2 lehrt, dass eine Anzeigeeinheit Daten von verschiedenen angeschlossenen elektromedizinischen Geräten empfängt und variabel darstellt. Dabei werden Alarminformationen in einem separaten expandierten Fenster eines Steuermenüs der Anzeigeeinheit dargestellt.

Aufgabe der vorliegenden Erfindung ist es, eine verbesserte Ausgabe eines elektromedizinischen Gerätes, insbesondere eine besonders sichere und präzise erfassbare Ausgabe von Daten mindestens eines elektromedizinischen Gerätes, bereitzustellen.

Erfindungsgemäß wird zur Lösung dieser Aufgabe ein Anzeigesystem zum Anzeigen einer Ausgabe mindestens eines elektromedizinischen Gerätes vorgeschlagen, mit einer Anzeigeeinheit, einer Anzeigesignaleinheit und einer Anzeigesignalüberwachungseinheit.

Die Anzeigeeinheit ist ausgebildet, ein Anzeigesignal zu empfangen und basierend auf dem Anzeigesignal eine visuelle Ausgabe darzustellen. Die visuelle Ausgabe erfolgt vorzugsweise über ein Display der Anzeigeeinheit.

Die Anzeigesignaleinheit weist eine Kommunikationsschnittstelle zur Kommunikation mit dem mindestens einen elektromedizinischen Gerät auf und ist ausgebildet, Daten, die das mindestens eine elektromedizinische Gerät betreffen, über die Kommunikationsschnittstelle zu empfangen. Vorzugsweise können Daten, die eine Mehrzahl von elektromedizinischen Gerät betreffen, beispielsweise die elektromedizinischen Geräte eines gemeinsamen Kommunikationsnetzwerkes, durch die Kommunikationsschnittstelle empfangen werden.

Dabei ist die Anzeigesignaleinheit ausgebildet, diese Daten einem Visualisierungstyp zuzuordnen, abhängig von den aktuell darzustellenden Visualisierungstypen eine jeweilige Darstellungsart der empfangenen Daten dynamisch zu bestimmen und basierend auf den Daten und der jeweils bestimmten Darstellungsart das Anzeigesignal zu erzeugen und an die Anzeigeeinheit auszugeben. Dabei umfasst die bestimmte Darstellungsart von Daten aus einer vorbestimmten Teilmenge von besonders relevanten Datenarten zumindest als Darstellungseigenschaft, dass eine Darstellung als für einen Nutzer erkennbare visuelle Ausgabe erfolgt. Weiterhin umfasst die bestimmte Darstellungsart als Darstellungseigenschaft eine Darstellungsposition innerhalb der visuellen Ausgabe der Anzeigeeinheit. Die Darstellungsposition kann eine feste Position innerhalb der visuellen Ausgabe beschreiben oder eine relative Position beschreiben, die eine Anordnung einer Darstellung der entsprechenden Daten relativ zu anderen im Rahmen der visuellen Ausgabe auszugebenden Daten festlegt. Die erkennbare visuelle Ausgabe stellt sicher, dass die Darstellung nicht angesichts anderer darzustellenden Daten unterbleibt, oder eine weitere Interaktion zwischen Nutzer und Anzeigeeinheit zum Erkennen der visuellen Ausgabe erfordert.

Die Anzeigesignalüberwachungseinheit ist mit der Anzeigeeinheit und der Anzeigesignaleinheit signaltechnisch verbunden und ist ausgebildet, das Anzeigesignal zu empfangen, insbesondere von der Anzeigesignaleinheit zu empfangen, und ein Ausgabesignal von der Anzeigeeinheit zu empfangen, wobei das Ausgabesignal Informationen zur erfolgten visuelle Ausgabe enthält. Dabei ist die Anzeigesignalüberwachungseinheit ausgebildet, Anzeigesignal und Ausgabesignal miteinander zu vergleichen und im Falle einer mangelnden Übereinstimmung zwischen erfolgter visueller Ausgabe und gemäß Anzeigesignal zu erfolgender visueller Ausgabe ein Warnsignal auszugeben, insbesondere ein Warnsignal an die Anzeigesignaleinheit auszugeben.

Im Rahmen der Erfindung wurde erkannt, dass bei der Verwendung eines gegenüber dem elektromedizinischen Gerät separaten Anzeigesystems sichergestellt werden muss, dass die empfangenen Daten auf geeignete Weise visuell ausgegeben werden. Hierbei wurde insbesondere erkannt, dass bestimmte besonders relevante Datenarten in jedem Fall ausgegeben werden sollten. Hierzu wird erfindungsgemäß eine Klassifizierung der empfangenen Daten in verschiedene Visualisierungstypen verwendet. Basierend auf den aktuell darzustellenden Visualisierungstypen kann dadurch eine jeweilige Darstellungsart dynamisch bestimmt werden.

Vorteilhaft kann hierdurch die visuelle Ausgabe an die aktuell empfangenen Daten angepasst werden. Insbesondere können besonders relevante Daten gegenüber weniger relevanten Daten deutlich hervorgehoben werden, beispielsweise durch eine entsprechende Wahl einer Schriftgröße der Darstellung und/oder einer Schriftart der Darstellung und/oder der Darstellungsposition.

Weiterhin wird durch die Überprüfung der visuellen Ausgabe durch die Anzeigesignalüberwachungseinheit sichergestellt, dass eine falsche oder schlecht erkennbare visuelle Ausgabe, beispielsweise aufgrund von Systemfehlern, durch das Anzeigesystem bemerkt wird. Durch die Ausgabe des Warnsignals kann das Anzeigesystem entsprechend reagieren und die visuelle Ausgabe umstellen oder zumindest den Nutzer des Anzeigesystems auf einen Fehler aufmerksam machen.

Besonders vorteilhaft sichert das erfindungsgemäße Anzeigesystem eine Kompatibilität mit verschiedenen elektromedizinischen Geräten, ohne dass eine aufwendige Abstimmung zwischen Anzeigeeinheit und einem jeweiligen elektromedizinischen Gerät stattfinden muss. Weiterhin ermöglicht das erfindungsgemäße Anzeigesystem besonders vorteilhaft die gleichzeitige visuelle Ausgabe von Daten mehrerer elektromedizinischer Geräte.

Ein weiterer Vorteil des erfindungsgemäßen Anzeigesystems besteht darin, dass sichergestellt werden kann, dass die Ausgabe von Daten nicht durch eine andere Ausgabe, wie beispielsweise durch ein Alarmsignal, verdeckt wird.

Die einzelnen Einheiten des erfindungsgemäßen Anzeigesystems können jeweils beabstandet voneinander oder innerhalb eines gemeinsamen Gehäuses angeordnet sein. Die Verarbeitung der Daten kann dabei vorteilhaft durch einen gemeinsamen Prozessor durchgeführt werden. Hierdurch wird die Verwendung mehrerer Prozessoren vermieden. Es kann jedoch auch die Funktionalität der Anzeigesignalüberwachungseinheit durch eine von der Anzeigesignaleinheit separate Einheit, d. h. zumindest durch einen separaten Prozessor durchgeführt werden.

Das erfindungsgemäße Anzeigesystem kann beabstandet von dem elektromedizinischen Gerät angeordnet sein.

Das Warnsignal ist erfindungsgemäß ein Signal, das einen entsprechenden Mangel an Übereinstimmung indiziert.

Das Empfangen des Anzeigesignals durch die Anzeigeeinheit schließt im Rahmen der Erfindung nicht aus, dass zusätzlich weitere Signale durch die Anzeigeeinheit empfangen werden.

Weiterhin erlaubt das erfindungsgemäße Anzeigesystem vorteilhaft die Erkennung einer Überlappung, falls neben dem Anzeigesignal noch Daten eines weiteren Signals, wie beispielsweise eines administrativen Anzeigesignals, durch die Anzeigeeinheit dargestellt werden und hierdurch Daten des Anzeigesignals teilweise nicht oder falsch dargestellt werden.

Das dynamische Bestimmen der jeweiligen Darstellungsart umfasst ein Bestimmen der Darstellungsart abhängig von den zugeordneten Visualisierungstypen entsprechend einer aktuell vorliegenden Anzahl an Visualisierungstypen. Somit erfolgt das dynamische Bestimmen nach dem Empfangen von Daten und einem entsprechenden Zuordnen eines Visualisierungstypen jeweils erneut. Dadurch liegt typischerweise kein fester Darstellungsort für einen bestimmten Visualisierungstyp vor, da dieser im Rahmen der dynamisch festgelegten Darstellungsart ebenfalls dynamisch festgelegt wird.

Eine mangelnde Übereinstimmung wird von der Anzeigesignalüberwachungseinheit festgestellt, falls die erfolgte visuelle Ausgabe ein vorbestimmtes Maß an Übereinstimmung unterschreitet. So liegt beispielsweise eine mangelnde Übereinstimmung vor, falls Daten aus der vorbestimmten Teilmenge von besonders relevanten Datenarten nicht in der für den Nutzer erkennbaren visuellen Ausgabe dargestellt werden. Dadurch wird vorteilhaft sichergestellt, dass besonders relevante Daten nicht durch administrative Daten, wie beispielsweise eine Anmeldemaske, eine Nutzerkennung, eine Patienteninformation und/oder eine Gerätekennung überdeckt werden. Das vorbestimmte Maß an Übereinstimmung kann sich ergänzend zum Inhalt der Daten auch auf eine visuelle Ähnlichkeit beziehen, beispielsweise bei einem Vergleich der Ähnlichkeit von anzuzeigender Schriftart und angezeigter Schriftart, oder auf die Erkennbarkeit von dargestellten Daten, beispielsweise durch eine Bewertung eines vorliegenden mangelnden Kontrastes zwischen einer Darstellungsfarbe und einer Hintergrundfarbe.

Die Gesamtheit aller Darstellungseigenschaften bildet die jeweilige Darstellungsart von empfangen Daten.

Bei den empfangenen Daten handelt es sich beispielsweise um physiologische Parameter, die durch das elektromedizinische Gerät bestimmt werden und/oder um eine Einstellung des elektromedizinischen Gerätes, wie etwa einen aktuellen Betriebsmodus, eine Alarmgrenze, eine Konfiguration und/oder ein aktuelles Patientenprofil, und/oder um einen Alarm, der durch das elektromedizinische Gerät bestimmt wurde.

Nachfolgend werden bevorzugte Ausführungsformen des erfindungsgemäßen Anzeigesystems beschrieben.

Eine mangelnde Übereinstimmung zwischen erfolgter visueller Ausgabe und zu erfolgender visueller Ausgabe liegt in einer Ausführungsform dann vor, wenn relevante klinische Daten durch eine überlagerte Anzeige von weiteren Daten nicht oder nur teilweise im Rahmen der visuellen Ausgabe dargestellt werden. In einer weiteren Ausführungsform wird durch diesen Vergleich von erfolgter visueller Ausgabe und zu erfolgender visueller Ausgabe sichergestellt, dass dargestellte Daten und insbesondere dargestellte Symbole von anderen Daten, wie etwa einer anderen Ziffer, einem anderen Buchstaben oder einem anderen Symbol unterschieden werden können.

Vorzugsweise erfolgt die visuelle Ausgabe über ein Display der Anzeigeeinheit. In einer besonders bevorzugten Variante handelt es sich bei dem Display um ein Touchdisplay. Vorzugsweise ist das Display umgeben von einem Gehäuse der Anzeigeeinheit.

Vorzugsweise ist die Anzeigesignalüberwachungseinheit ausgebildet, das Warnsignal an die Anzeigesignaleinheit auszugeben. Hierdurch kann ein Fehler bei der Anzeige der Daten durch die Anzeigeeinheit direkt durch die Anzeigesignaleinheit behoben werden. In einer Variante dieser Ausführungsform ist die Anzeigesignaleinheit dazu ausgebildet, ein Fehlersignal an einen Nutzer des Anzeigesystems auszugeben, falls der durch das Warnsignal indizierte Fehler nicht behoben werden kann. Eine solche Ausgabe an den Nutzer kann beispielsweise durch eine Darstellung einer Fehlermeldung über die Anzeigeeinheit realisiert sein.

In einer Ausführungsform ist die Anzeigesignaleinheit weiterhin ausgebildet, abhängig von dem empfangenen Warnsignal eine Anpassung der jeweiligen dynamisch festgelegten Darstellungsart auszuführen. Hierdurch kann sichergestellt werden, dass wieder eine Übereinstimmung zwischen der visuellen Ausgabe und der zu erfolgenden visuellen Ausgabe erreicht wird.

In einer zur vorherigen Ausführungsform alternativen und/oder ergänzenden Ausführungsform löst das Warnsignal eine Fehlermeldung aus, die über das Anzeigesignal durch die Anzeigeeinheit visuell ausgegeben wird. Hierdurch wird ein Nutzer des erfindungsgemäßen Anzeigesystems darüber informiert, dass derzeit ein Fehler des Anzeigesystems vorliegt. Das visuelle Ausgeben der Fehlermeldung erfolgt beispielsweise durch eine für einen Nutzer erkennbare Veränderung der Darstellungsart, wie beispielsweise durch ein Ausgrauen eines bestimmten Bereichs der visuellen Ausgabe.

In einer bevorzugten Ausführungsform umfasst die vorbestimmte Teilmenge von besonders relevanten Datenarten zumindest Alarminformationen und/oder klinische Messwerte. Hierdurch wird sichergestellt, dass zumindest derartige besonders relevante Daten tatsächlich durch die Anzeigeeinheit visuell ausgegeben werden. Dies ist insbesondere dann vorteilhaft, wenn eine große Anzahl an Daten durch das Anzeigesystem empfangen wird, sodass nicht alle Daten gleichzeitig ausgegeben werden können. Vorzugsweise indiziert der zugeordnete Visualisierungstyp bereits eine Priorisierungsinformation, die angibt, gegenüber welchen anderen Daten eine Priorisierung der Daten dieses Visualisierungstyps erfolgen soll. Die Priorisierungsinformationen kann ein Relevanzlevel sein, das durch einen Vergleich mit dem Relevanzlevel von anderen Daten anzeigt, ob diese Daten gegenüber den anderen Daten priorisiert sind oder nicht.

In einer weiteren vorteilhaften Ausführungsform ist die Anzeigesignaleinheit weiterhin ausgebildet, das Anzeigesignal derart zu erzeugen, dass ein entsprechender Hinweis über die visuelle Ausgabe, insbesondere die graphische Ausgabe, dargestellt wird, falls aufgrund der Dimensionierung der visuellen Ausgabe, insbesondere der graphischen Ausgabe, nicht alle von dem elektromedizinischen Gerät empfangenen Daten durch die Anzeigeeinheit visuell ausgegeben werden können. Hierdurch wird ein Nutzer des erfindungsgemäßen Anzeigesystems darüber informiert, dass nicht alle empfangenen Daten über die Anzeigeeinheit dargestellt werden. In einer bevorzugten Variante diese Ausführungsform können nicht dargestellte Daten durch eine Interaktion zwischen Nutzer und Anzeigeeinheit dargestellt werden. Eine derartige Interaktion ist beispielsweise das Berühren einer entsprechenden Steuerfläche auf einem Touchdisplay der Anzeigeeinheit. In einem weiteren Beispiel dieser Variante ist eine derartige Interaktion das Nutzen einer Scroll-Leiste.

In einer weiteren vorteilhaften Ausführungsform ist die Anzeigesignaleinheit weiterhin ausgebildet, eine Darstellungsgröße und/oder die Darstellungsposition von empfangenen Daten eines Visualisierungstyps abhängig von einem diesem Visualisierungstyp zugeordneten Relevanzlevel zu bestimmen. In dieser Ausführungsform werden vorteilhaft besonders relevante Informationen anders dargestellt als weniger relevante Informationen. Insbesondere können besonders relevante Informationen durch eine größere Schriftgröße und/oder durch eine besonders zentrale Position innerhalb der visuellen Ausgabe dargestellt werden. Durch die Verwendung verschiedener Darstellungsgrößen kann eine Anzahl an dargestellten Daten erhöht werden. Insbesondere können weniger relevante Informationen durch eine kleinere Schriftgröße dargestellt werden als relevante Informationen. Hierdurch kann eine große Anzahl von weniger relevanten Informationen gleichzeitig dargestellt werden, ohne dass dadurch die Darstellung von relevanten Informationen beeinträchtigt wird. In einer alternativen und/oder ergänzenden Ausführungsform umfasst die Darstellungsart als Darstellungseigenschaft neben der Darstellungsgröße und der Darstellungsposition einen Darstellungsstil. Der Darstellungsstil gibt beispielsweise eine zu nutzende Schriftart und/oder eine zu nutzende Schriftfarbe der darzustellenden Information an. Als Information ist hier und im Weiteren die jeweils durch empfangene Daten indizierte Information zu verstehen.

In einer bevorzugten Ausführungsform weist das Anzeigesystem weiterhin eine Eingabeeinheit auf, die mit der Anzeigesignaleinheit signaltechnisch verbunden ist und eine Benutzerschnittstelle aufweist. Dabei ist die Eingabeeinheit ausgebildet, über die Benutzerschnittstelle abhängig von einer Benutzereingabe eines Nutzers des Anzeigesystems ein Eingabesignal ausgeben. Weiterhin ist die Anzeigesignaleinheit ausgebildet, das Eingabesignal zu empfangen und abhängig von dem dem Eingabesignal zugeordneten Visualisierungstyp eine entsprechende Darstellungsart zu bestimmen und ein Anzeigesignal basierend auf allen aktuell empfangenen Daten und der jeweils bestimmten Darstellungsart zu erzeugen und auszugeben. Die Benutzereingabe kann beispielsweise eine Berechtigungsinformation aufweisen, wie etwa ein Passwort. Weiterhin kann die Benutzereingabe eine die empfangenen Daten betreffende Information aufweisen, wie etwa eine personenspezifische Information. Die Benutzerschnittstelle kann eine Tastatur, eine Computermaus, ein Touch-Bedienelement oder ein Joystick sein.

In einer besonders bevorzugten Ausführungsform ist die Anzeigesignalüberwachungseinheit weiterhin ausgebildet, eine sich in zeitlichen Abständen ändernde visuelle Signatur über ein Signatursignal an die Anzeigesignaleinheit auszugeben. Dabei ist die Anzeigesignaleinheit weiterhin ausgebildet, die aktuelle visuelle Signatur über das Anzeigesignal auszugeben, und die Anzeigesignalüberwachungseinheit ist weiterhin ausgebildet, über das Ausgabesignal die aktuell dargestellte visuelle Signatur mit der aktuellen visuellen Signatur zu vergleichen und abhängig von diesem Vergleich eine Prüfinformation bereitzustellen. In dieser Ausführungsform wird vorteilhaft eine Funktionsfähigkeit der Anzeigesignaleinheit überprüft. So zeigt eine Übereinstimmung zwischen der aktuell dargestellten visuellen Signatur und der an die Anzeigesignaleinheit ausgegebenen anzuzeigenden visuellen Signatur an, dass die Anzeigesignaleinheit empfangene Daten weiterverarbeiten kann. Insbesondere bei der Anwendung im medizinischen Umfeld wird hierdurch vorteilhaft sichergestellt, dass neu empfangene Daten, die möglicherweise eine Veränderung von Messwerten eines elektromedizinischen Gerätes anzeigen, weiterhin verarbeitet werden. Durch die Verwendung der sich ändernden visuellen Signatur kann sichergestellt werden, dass ein Fehler bei der Verarbeitung von Daten besonders schnell erkannt wird. In einer vorteilhaften Variante diese Ausführungsform sind die zeitlichen Abstände regelmäßige zeitliche Abstände, insbesondere zeitliche Abstände von weniger als 30 Sekunden, vorzugsweise weniger als 20 Sekunden, besonders bevorzugt weniger als 10 Sekunden.

In einer weiteren bevorzugten Ausführungsform ist die Anzeigeeinheit weiterhin ausgebildet, ein zusätzliches administratives Anzeigesignal zu empfangen und basierend auf dem Anzeigesignal und dem zusätzlichen administrativen Anzeigesignal eine kombinierte visuelle Ausgabe darzustellen, wobei das zusätzliche administrative Anzeigesignal nicht-medizinische Daten eines externen Gerätes indiziert. Hierbei kann die Darstellung der nicht-medizinischen Daten die Darstellung der Daten des Anzeigesignals verhindern oder zumindest beeinflussen. In diesem Ausführungsbeispiel stellt die Anzeigesignalüberwachungseinheit vorteilhaft sicher, dass die Daten entsprechend ihres zugeordneten Visualisierungstyps in jedem Fall dargestellt werden, wenn sie zu der vorbestimmten Teilmenge von besonders relevanten Datenarten gehören. So würde beispielsweise im Falle einer Überlagerung dieser Daten durch die nicht-medizinischen Daten die Anzeigesignalüberwachungseinheit eine mangelnde Übereinstimmung zwischen erfolgter visueller Ausgabe und zu erfolgender visueller Ausgabe feststellen und ein entsprechendes Warnsignal ausgeben. Nicht-medizinische Daten können beispielsweise eine separat über das administrative Anzeigesignal empfangene Patienteninformation, eine Gerätekennungen, eine Nutzerkennung und/oder eine Anmeldemaske zur Eingabe einer Benutzerkennung sein.

In einer besonders bevorzugten Ausführungsform des erfindungsgemäßen Anzeigesystems ist die Kommunikationsschnittstelle ausgebildet, Daten von einer Mehrzahl von elektromedizinischen Geräten zu empfangen. Vorzugsweise umfasst der hierbei den Daten durch die Anzeigesignaleinheit zugeordnete Visualisierungstyp eine Geräteinformation, die das zu den Daten korrespondierende elektromedizinische Gerät indiziert. In dieser Ausführungsform wird besonders vorteilhaft ausgenutzt, dass das erfindungsgemäß Anzeigesystem separat von dem elektromedizinischen Gerät funktioniert, dessen Ausgabe durch das Anzeigesystem bereitgestellt werden soll. In einer Variante diese Ausführungsform umfasst der diesen Daten zugeordnete Visualisierungstyp ein Relevanzlevel, das von dem zu diesen Daten korrespondierenden elektromedizinischen Gerät abhängig ist.

Vorzugsweise empfängt die Anzeigesignaleinheit über die Kommunikationsschnittstelle sämtliche Daten von elektromedizinischen Geräten innerhalb eines elektromedizinischen Kommunikationsnetzwerks. Ein solches elektromedizinisches Kommunikationsnetzwerk ist dem jeweiligen Anzeigesystem zugeordnet und umfasst typischerweise eine Mehrzahl von elektromedizinischen Geräten. In einem ersten Beispiel umfasst das elektromedizinische Kommunikationsnetzwerk sämtliche elektromedizinische Geräte, die für die Behandlung eines einzigen konkreten Patienten verwendet werden. In einem zweiten Beispiel umfasst das elektromedizinische Kommunikationsnetzwerk sämtliche elektromedizinischen Geräte einer einzigen Geräteart, die auf einer Krankenhausstation verwendet werden.

Die Kommunikationsschnittstelle der erfindungsgemäßen Anzeigesignaleinheit erlaubt vorzugsweise eine kabellose Übertragung von Daten. In einer alternativen und/oder ergänzenden Ausführungsform erlaubt die Kommunikationsschnittstelle eine Kabel-basierte Übertragung von Daten. In einer weiteren Ausführungsform erlaubt die Kommunikationsschnittstelle sowohl eine kabellose Übertragung von Daten als auch eine Kabel-basierte Übertragung von Daten.

In einer weiteren Ausführungsform ist die Anzeigeeinheit beabstandet von der Anzeigesignaleinheit und der Anzeigesignalüberwachungseinheit ausgebildet. In einer Variante dieser Ausführungsform sind die Anzeigesignaleinheit und die Anzeigesignalüberwachungseinheit in einem gemeinsamen Gehäuse angeordnet und mit der Anzeigeeinheit signaltechnisch verbunden.

Gemäß einem weiteren Aspekt der Erfindung wird die oben genannte Aufgabe gelöst durch ein Verfahren zum Anzeigen einer Ausgabe mindestens eines elektromedizinischen Gerätes.

Das erfindungsgemäße Verfahren weist die folgenden Schritte auf:
- Empfangen von Daten, die das mindestens eine elektromedizinische Gerät betreffen;
- Zuordnen der empfangenen Daten zu einem jeweiligen Visualisierungstyp und dynamisches Bestimmen einer jeweiligen Darstellungsart der empfangenen Daten basierend auf den aktuell darzustellenden Visualisierungstypen;
- Erzeugen und Ausgeben eines Anzeigesignals basierend auf den empfangen Daten und der jeweils bestimmten Darstellungsart, wobei die bestimmte Darstellungsart von Daten aus einer vorbestimmten Teilmenge von besonders relevanten Datenarten zumindest als Darstellungseigenschaft umfasst, dass eine Darstellung als für einen Nutzer erkennbare visuelle Ausgabe erfolgt, und wobei die bestimmte Darstellungsart weiterhin als Darstellungseigenschaft eine Darstellungsposition umfasst;
- Empfangen des Anzeigesignals und Darstellen einer visuellen Ausgabe basierend auf dem Anzeigesignal;
- Senden eines Ausgabesignals, das Informationen zur erfolgten visuellen Ausgabe enthält;
- Empfangen des Anzeigesignals und des Ausgabesignals und Vergleichen der beiden Signale miteinander;
- Ausgeben eines Warnsignals, falls eine mangelnde Übereinstimmung zwischen erfolgter visueller Ausgabe und gemäß Anzeigesignal zu erfolgender visueller Ausgabe vorliegt.

Das Verfahren gemäß dem weiteren Aspekt der Erfindung erlaubt besonders vorteilhaft die Verwendung eines separaten Gerätes für das Anzeigen von Daten eines elektromedizinischen Gerätes. Weiterhin wird vorteilhaft die Anzeige von Daten verschiedener elektromedizinische Geräte durch ein einziges Anzeigemedium ermöglicht. Hierdurch kann eine besonders große Übersichtlichkeit der medizinischen Daten sichergestellt werden.

Weiterhin erlaubt das erfindungsgemäße Verfahren vorteilhaft eine andauernde Überprüfung der Ausgabe der Daten, sodass ein Fehler bei dieser Ausgabe schnell erkannt werden kann. Hierdurch ist das erfindungsgemäße Verfahren zur Anzeige von Daten besonders sicher und ist besonders wenig anfällig für Fehler.

Weiterhin erlaubt das erfindungsgemäße Verfahren die Erkennung einer Überlappung, falls neben dem Anzeigesignal noch Daten eines weiteren Signals, wie beispielsweise eines administrativen Anzeigesignals, durch die Anzeigeeinheit dargestellt werden und hierdurch Daten des Anzeigesignals teilweise nicht oder falsch dargestellt werden.

Nachfolgend werden bevorzugte Ausführungsformen des erfindungsgemäßen Verfahrens beschrieben.

In einer vorteilhaften Ausführungsform des erfindungsgemäßen Verfahrens erfolgt das Erzeugen des Anzeigesignals derart, dass ein entsprechender Hinweis über die visuelle Ausgabe dargestellt wird, falls aufgrund der Dimensionierung der visuelle Ausgabe nicht alle von dem elektromedizinischen Gerät empfangenen Daten visuell ausgegeben werden können. Hierdurch wird ein Anwender des Verfahrens darüber informiert, dass aktuell mehr Daten empfangen werden als gleichzeitig ausgegeben werden können.

In einer weiteren bevorzugten Ausführungsform weist das Verfahren weiterhin die Schritte auf:
- Ausgeben von einer sich in zeitlichen Abständen ändernden visuellen Signatur über ein Signatursignal;
- Empfangen des Signatursignals und Ausgeben der aktuellen visuellen Signatur über das Anzeigesignal;
- Vergleichen der aktuell dargestellten visuellen Signatur aus dem Ausgabesignal mit der aktuellen visuellen Signatur und Bereitstellen einer Prüfinformation abhängig von diesem Vergleich.
Durch das andauernde Vergleichen der aktuell dargestellten visuellen Signatur mit der aktuell darzustellenden visuellen Signatur, wird ein Fehler bei der Darstellung von Daten besonders schnell erkannt. Dies ist insbesondere dann vorteilhaft, wenn frühere Daten weiterhin dargestellt werden, ohne jedoch neu empfangene Daten bei dieser Darstellung zu berücksichtigen. In einem solchen Fehlerfall würde ohne die Überprüfung mittels visueller Signatur die Gefahr bestehen, dass ein Fehler bei der Ausgabe erst spät erkannt wird.

In einer besonders bevorzugten Ausführungsform ist das Verfahren zum Anzeigen einer Ausgabe einer Mehrzahl von elektromedizinischen Geräten ausgestaltet. In dieser Ausführungsform umfasst das Verfahren den zusätzlichen Schritt:
- Empfangen von Daten , die mindestens ein weiteres elektromedizinisches Gerät betreffen, wobei der den Daten zugeordnete Visualisierungstyp eine Geräteinformation umfasst, die das zu den Daten korrespondierende elektromedizinische Gerät indiziert.
In dieser Ausführungsform wird vorteilhaft ausgenutzt, dass das erfindungsgemäße Verfahren unabhängig von dem jeweiligen elektromedizinischen Gerät ausgeführt werden kann. Hierdurch ist es insbesondere möglich, dass Daten von der Mehrzahl von elektromedizinischen Geräten gleichzeitig durch das erfindungsgemäße Verfahren angezeigt werden können. Durch die jeweilige Geräteinformation lässt sich vorzugsweise auf ein Relevanzlevel der empfangenen Daten schließen.

Weiterhin wird die oben genannte Aufgabe gelöst durch ein Programm mit einem Programmcode zur Durchführung des Verfahrens gemäß mindestens einer der oben angegebenen Ausführungsformen des erfindungsgemäßen Verfahrens, wenn der Programmcode auf einem Computer, einem Prozessor oder einer programmierbaren Hardwarekomponente ausgeführt wird.

Das Programm kann dabei auch nur einen Teil der erfindungsgemäßen Datenverarbeitung ausführen. Vorzugsweise werden zumindest die Funktion der Anzeigesignaleinheit und der Anzeigesignalüberwachungseinheit durch ein Programm und/oder aufeinander abgestimmte Teile eines Programms gesteuert. Vorzugsweise wird das erfindungsgemäße Programm von einem Prozessor des Anzeigesystems ausgeführt. Alternativ wird das Programm zumindest durch einen ersten Prozessor der Anzeigesignaleinheit und durch einen zweiten Prozessor der Anzeigesignalüberwachungseinheit ausgeführt.

Die Erfindung soll nun anhand von in den Figuren schematisch dargestellten, vorteilhaften Ausführungsbeispielen näher erläutert werden. Von diesen zeigen im Einzelnen:
- Fig. 1: eine schematische Darstellung eines ersten Ausführungsbeispiels eines erfindungsgemäßen Anzeigesystems;
- Fig. 2: eine schematische Darstellung eines zweiten Ausführungsbeispiels des erfindungsgemäßen Anzeigesystems;
- Fig. 3: eine schematische Darstellung eines dritten Ausführungsbeispiels des erfindungsgemäßen Anzeigesystems;
- Fig. 4: eine schematische Darstellung eines ersten Ausführungsbeispiels einer visuellen Ausgabe des erfindungsgemäßen Anzeigesystems;
- Figs. 5, 6: eine schematische Darstellung der visuellen Ausgabe des erfindungsgemäßen Anzeigesystems für die Anzeige eines Alarmzustandes (Fig. 5) und für die Anzeige einer nicht vollständigen Ausgabe von empfangenen Daten (Fig. 6);
- Fig. 7: eine schematische Darstellung der visuellen Ausgabe des erfindungsgemäßen Anzeigesystems für eine Überlappung der visuellen Ausgabe durch ein administratives Anzeigesignal;
- Fig. 8: ein Flussdiagramm eines ersten Ausführungsbeispiels eines erfindungsgemäßen Verfahrens.

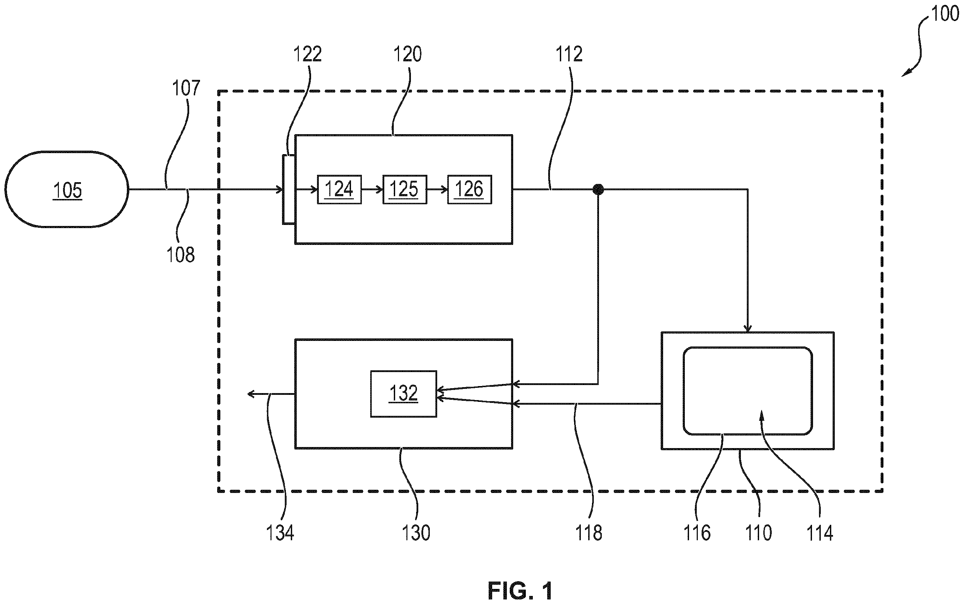

Fig. 1 zeigt eine schematische Darstellung eines ersten Ausführungsbeispiels eines erfindungsgemäßen Anzeigesystems 100.

Das Anzeigesystem 100 ist zum Anzeigen einer Ausgabe mindestens eines elektromedizinischen Gerätes 105 ausgebildet. Das Anzeigesystem 100 umfasst dazu eine Anzeigeeinheit 110, eine Anzeigesignaleinheit 120 und eine Anzeigesignalüberwachungseinheit 130.

Die Anzeigeeinheit 110 ist ausgebildet, ein Anzeigesignal 112 zu empfangen und basierend auf dem Anzeigesignal 112 eine visuelle Ausgabe 114 darzustellen. Die visuelle Ausgabe 114 erfolgt auf einem Display 116 der Anzeigeeinheit 110. Die Anzeigesignaleinheit 120 weist eine Kommunikationsschnittstelle 122 zur Kommunikation mit dem elektromedizinischen Gerät 105 auf und ist ausgebildet, Daten 107, die das elektromedizinische Gerät 105 betreffen, über die Kommunikationsschnittstelle 122 zu empfangen. Hierfür empfängt die Kommunikationsschnittstelle 122 das die Daten 107 indizierende Gerätesignal 108. Vorliegend erfolgt der Empfang des Gerätesignals 108 Kabel-basiert über eine Ethernet-Verbindung. In einem nicht dargestellten Ausführungsbeispiel erfolgt der Empfang des Gerätesignals über eine kabellose Verbindung, insbesondere über WLAN, Bluetooth, BLE oder ZigBee. In einem weiteren nicht dargestellten Ausführungsbeispiel wird die Kabel-basierte Verbindung durch ein Bussystem, wie etwa durch ein USB-System gebildet.

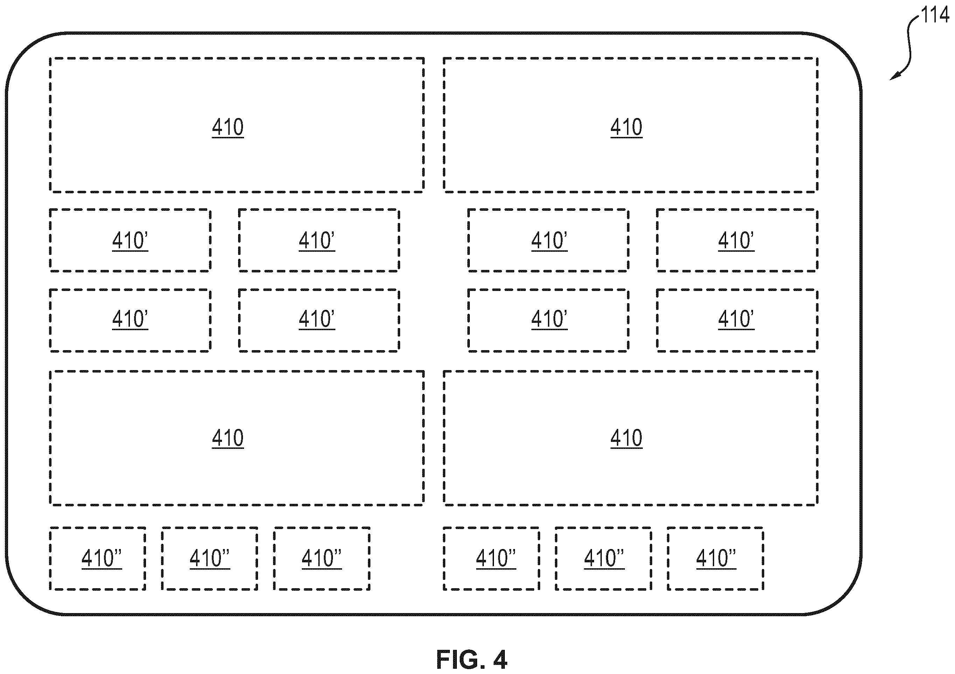

Im Rahmen eines ersten Verarbeitungsschrittes 124 ist die Anzeigesignaleinheit 120 ausgebildet, die Daten 107 einem Visualisierungstyp zuzuordnen. In einem darauffolgenden zweiten Verarbeitungsschritt 125 wird abhängig von den aktuell darzustellenden Visualisierungstypen eine jeweilige Darstellungsart der empfangenen Daten 107 dynamisch bestimmt. Basierend auf den Daten 107 und der jeweils bestimmten Darstellungsart wird in einem darauffolgenden dritten Verarbeitungsschritt 126 der Anzeigesignaleinheit 120 das Anzeigesignal 112 erzeugt und an die Anzeigeeinheit 110 ausgegeben. Dabei umfasst die bestimmte Darstellungsart von Daten aus einer vorbestimmten Teilmenge von besonders relevanten Datenarten zumindest als Darstellungseigenschaft die Vorgabe, dass eine Darstellung als für einen Nutzer erkennbare visuelle Ausgabe erfolgt. Dies bedeutet, dass auch dann eine Ausgabe für diese vorbestimmte Teilmenge erfolgt, wenn aktuell nicht alle Daten ausgegeben werden können. Mithin hat die Darstellung dieser vorbestimmten Teilmenge eine besonders hohe Priorität, sodass deren Darstellung durch die Anzeigesignaleinheit 120 sichergestellt ist. Weiterhin umfasst die bestimmte Darstellungsart grundsätzlich als Darstellungseigenschaft eine Darstellungsposition. Die Darstellungsposition beschreibt die Position der Darstellung der entsprechenden Daten im Rahmen der visuellen Ausgabe der Anzeigeeinheit 110. Beispiele für die Darstellungsposition und für die Darstellungseigenschaft sind im Rahmen der Figuren 4 bis 6 beschrieben.

Das ausgegebene Anzeigesignal 112 wird erfindungsgemäß sowohl an die Anzeigeeinheit 110 als auch an die Anzeigesignalüberwachungseinheit 130 ausgegeben. Dabei können sich das an die Anzeigeeinheit 110 und das an die Anzeigesignalüberwachungseinheit 130 ausgegebene Anzeigesignal 112 voneinander unterscheiden, jedoch umfassen beide Anzeigesignale erfindungsgemäß als zueinander korrespondierenden Signalanteil die empfangenen Daten und die bestimmte Darstellungsart.

Die Anzeigesignalüberwachungseinheit 130 ist mit der Anzeigeeinheit 110 und der Anzeigesignaleinheit 120 signaltechnisch verbunden. Vorliegend ist diese Verbindung Kabel-basiert ausgebildet. Vorzugsweise verfügen Anzeigesignalüberwachungseinheit 130 und Anzeigeeinheit 110 über einen gemeinsam genutzten Speicherbereich. In einem nicht dargestellten Ausführungsbeispiel wird das Anzeigesignal über eine Funkverbindung übertragen.

Die Anzeigesignalüberwachungseinheit 130 ist ausgebildet, das Anzeigesignal von der Anzeigesignaleinheit 120 zu empfangen und ein Ausgabesignal 118 von der Anzeigeeinheit 110 zu empfangen. Das Ausgabesignal 118 enthält Informationen zur erfolgten visuelle Ausgabe 114 der Anzeigeeinheit 110. Dadurch erlaubt das Ausgabesignal 118 eine Auswertung, wie die empfangenen und verarbeiteten Daten 107 im Rahmen der visuellen Ausgabe 114 dargestellt wurden. Die Anzeigesignalüberwachungseinheit 130 ist dabei ausgebildet, im Überwachungsschritt 132 basierend auf dieser Auswertung der visuellen Ausgabe 114 und der zu erfolgenden visuellen Ausgabe einen Vergleich zwischen Anzeigesignal 112 und Ausgabesignal 118 durchzuführen. Bei mangelnder Übereinstimmung zwischen erfolgter visueller Ausgabe 114 und zu erfolgender visueller Ausgabe ist die Anzeigesignalüberwachungseinheit 130 weiterhin ausgebildet ein Warnsignal 134 auszugeben.

Das Warnsignal 134 wird vorliegend an einen Nutzer des Anzeigesystems 100 als optisches Signal ausgegeben. Zusätzlich oder alternativ wird das Warnsignal in einem Ausführungsbeispiel an die Anzeigesignaleinheit ausgegeben. Hierdurch kann die Anzeigesignaleinheit automatisiert eine Korrektur des Anzeigesignals durchführen.

Die vorbestimmte Teilmenge von besonders relevanten Daten umfasst zumindest Alarminformationen und/oder klinische Messwerte des mindestens einen verbundenen elektromedizinischen Gerätes. Hierdurch wird sichergestellt, dass ein Alarm in jedem Fall dargestellt wird, auch wenn aktuell nicht alle empfangenen Daten auf dem Display 116 dargestellt werden können. So wird sichergestellt, dass ein Nutzer des Anzeigesystems 100 über einen Alarm informiert wird.

Der Vergleich von erfolgter visueller Ausgabe 114 und zu erfolgender visueller Ausgabe umfasst erfindungsgemäß auch eine Prüfung durch die Anzeigesignalüberwachungseinheit 130, ob alle darzustellenden Buchstaben, Ziffern und/oder Symbole für den Nutzer erkennbar sind. So wird erkannt, ob eine verwendete Schriftart für die Darstellung der Daten aktuell ungeeignet ist, ob Daten durch eine Ausgabe anderer Daten vollständig oder teilweise überdeckt werden und/oder ob eine Schriftfarbe für die Darstellung der Daten aktuell ungeeignet ist.

Bei den empfangen Daten 107 handelt es sich vorzugsweise um physiologische Parameter, die durch das mindestens eine elektromedizinische Gerät 105 bestimmt wurden und/oder um eine Einstellung des elektromedizinischen Gerätes 105 und/oder um einen Alarm, der durch das elektromedizinische Gerät 105 bestimmt wurde. Bei den bestimmten physiologischen Parametern kann es sich beispielsweise um Messwerte handeln, wie etwa den invasiven oder nichtinvasiven Blutdruck, die Herzfrequenz, die Pulsrate, die Sauerstoffsättigung, die Körpertemperatur und/oder die Arrhythmie-Episoden. Bei den Einstellungen des elektromedizinischen Gerätes 105 kann es sich beispielsweise um eine Alarmgrenze, einen Betriebsmodus, einen erkannten Alarmzustand und/oder einen Signalisierungszustand handeln.

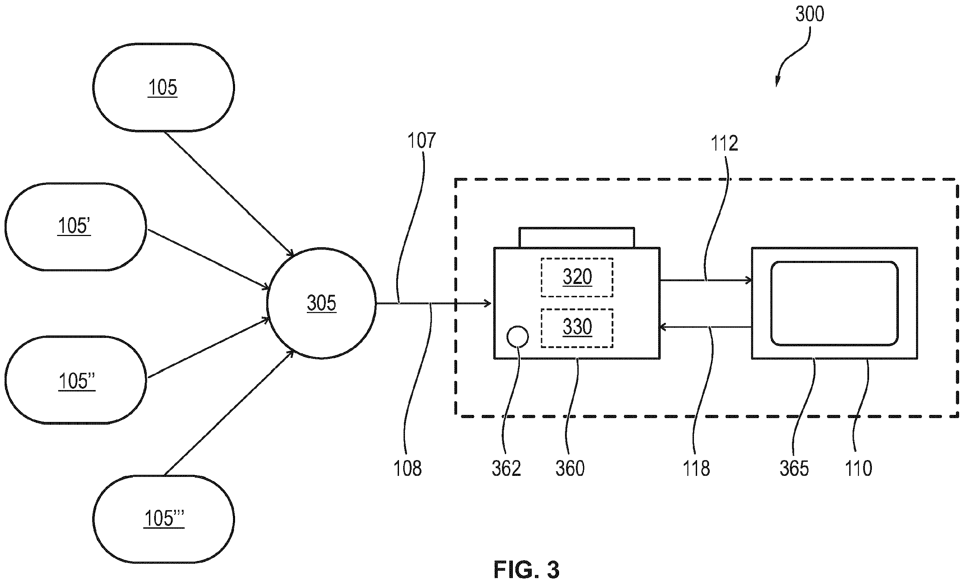

In dem dargestellten Ausführungsbeispiel sind sämtliche Einheiten 110, 120, 130 des Anzeigesystems 100 in einem gemeinsamen Gehäuse angeordnet. In anderen Ausführungsbeispielen sind diese Einheiten in getrennten Gehäusen angeordnet, wie im Rahmen von Fig. 3 dargestellt.

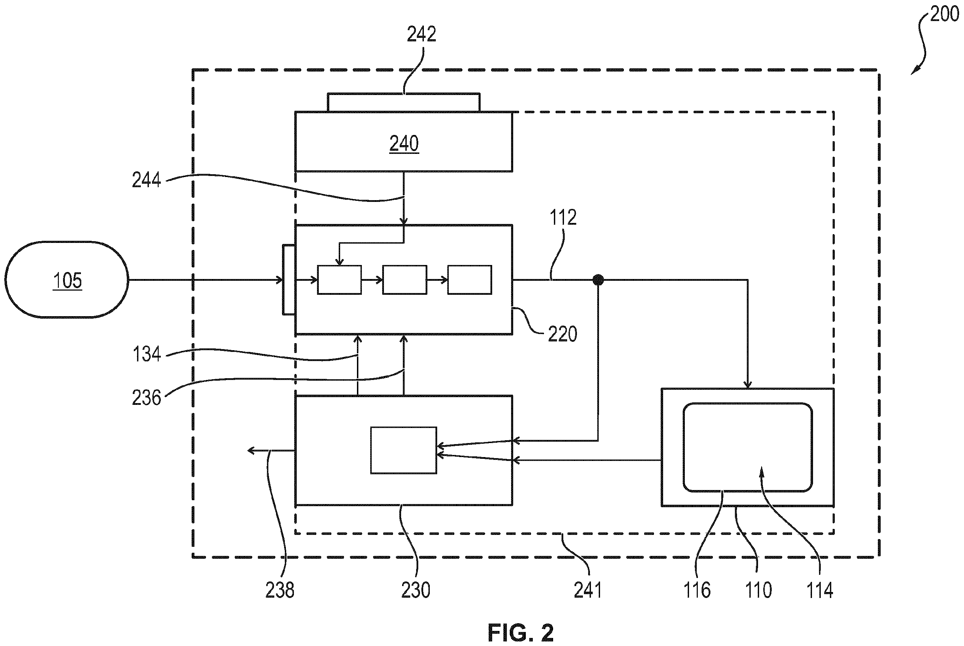

Fig. 2 zeigt eine schematische Darstellung eines zweiten Ausführungsbeispiels des erfindungsgemäßen Anzeigesystems 200.

Das dargestellte Anzeigesystem 200 unterscheidet sich von dem in Fig. 1 gezeigten Anzeigesystem 100 dadurch, dass es eine Eingabeeinheit 240 aufweist.

Die Eingabeeinheit 240 ist mit der Anzeigesignaleinheit 220 signaltechnisch verbunden. Vorliegend handelt es sich hierbei um eine Kabel-basierte Verbindung. In einem nicht dargestellten Ausführungsbeispiel handelt es sich um eine kabellose Verbindung. Die Eingabeeinheit 240 weist weiterhin eine Benutzerschnittstelle 242 auf, über die eine Benutzereingabe eines Nutzers des Anzeigesystems 200 empfangen werden kann. Vorliegend handelt es sich bei der Benutzerschnittstelle 242 um eine Tastatur. In einem nicht dargestellten Ausführungsbeispiel handelt sich bei der Benutzerschnittstelle um ein Touch-Bedienelement, eine Computermaus oder um einen Joystick. Die Eingabeeinheit 240 ist dabei ausgebildet, abhängig von der Benutzereingabe ein Eingabesignal 244 auszugeben.

Die Anzeigesignaleinheit 220 ist in dem vorliegend dargestellten Ausführungsbeispiel dazu ausgebildet, das Eingabesignal 244 direkt über die Kabel-basierte Verbindung zu empfangen und abhängig von dem dem Eingabesignal 244 zugeordneten Visualisierungstyp eine entsprechende Darstellungsart zu bestimmen. Über das Anzeigesignal 112 werden durch die Benutzereingabe indizierte Daten entsprechend der bestimmten Darstellungsart ausgegeben und im Rahmen der visuellen Ausgabe 114 dargestellt.

Die Benutzereingabe kann vorliegend beispielsweise eine Konfiguration des mindestens einen verbundenen elektromedizinischen Gerätes 105 umfassen, beispielsweise eine Konfiguration von Daten einer klinischen Akte oder eine Konfiguration von Daten des elektromedizinischen Gerätes 105.

Weiterhin unterscheidet sich das Anzeigesystem 200 von dem in Fig. 1 dargestellten Anzeigesystem 100 dadurch, dass die Anzeigesignalüberwachungseinheit 230 dazu ausgebildet ist, das Warnsignal 134 an die Anzeigesignaleinheit 220 auszugeben. Die Anzeigesignaleinheit 220 ist dabei weiterhin ausgebildet, das Warnsignal 134 zu empfangen und einen darin aufgezeigten Fehler automatisiert zu korrigieren. Dies kann beispielsweise durch eine automatisierte Änderung einer Darstellungseigenschaft und mithin der bestimmten Darstellungsart erfolgen. Beispielsweise kann eine automatisierte Änderung einer Schriftfarbe für eine bessere visuelle Erkennbarkeit von Daten notwendig sein. In einem weiteren Beispiel ist eine Anpassung der Schriftart notwendig, um verschiedene Buchstaben, Ziffern und/oder Symbole voneinander unterscheiden zu können.

Weiterhin ist das Anzeigesystem 200 zusätzlich dazu ausgebildet eine Überprüfung seiner eigenen Funktionsfähigkeit durch die Nutzung einer visuellen Signatur auszuführen. Hierzu ist die Anzeigesignalüberwachungseinheit 230 dazu ausgebildet, die sich in regelmäßigen zeitlichen Abständen ändernde vorbestimmte visuelle Signatur über ein Signatursignal 236 an die Anzeigesignaleinheit 220 auszugeben. Die vorbestimmte visuelle Signatur ist vorzugsweise eine Kombination von Buchstaben, Ziffern und/oder Symbolen. Die Anzeigesignaleinheit 224 ist weiterhin ausgebildet, die aktuell empfangene visuelle Signatur über das Anzeigesignal 112 auszugeben. Die Anzeigeeinheit 110 wird die visuelle Signatur im Rahmen der visuellen Ausgabe 114 darstellen. Die Anzeigesignalüberwachungseinheit 230 kann daher im regulären Betrieb die dargestellte visuelle Signatur mit der aktuell darzustellenden Signatur vergleichen und abhängig von diesem Vergleich eine Prüfinformation bereitstellen. Dies erfolgt vorliegend durch die Ausgabe eines Prüfsignals 238.

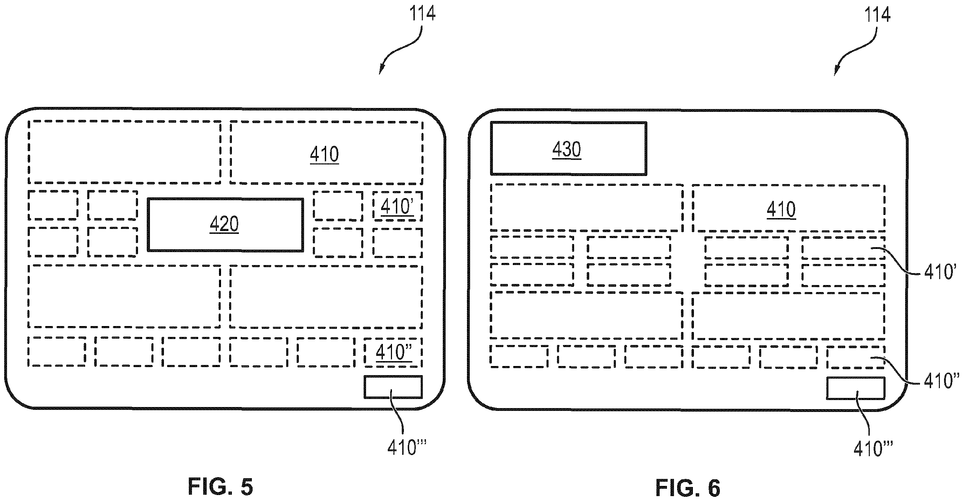

Ein Beispiel für die Darstellung der visuellen Signatur ist im Rahmen von Fig. 5 und 6 gezeigt.

In dem dargestellten Ausführungsbeispiel ist die Eingabeeinheit 240 derart in einem Gesamtgehäuse 241 des Anzeigesystems 200 angeordnet, dass die Benutzerschnittstelle 242 an dem Gehäuse angeordnet ist und das Gehäuse das Display 116 umgibt.

Fig. 3 zeigt eine schematische Darstellung eines dritten Ausführungsbeispiels des erfindungsgemäßen Anzeigesystems 300.

Das Anzeigesystem 300 unterscheidet sich von den in Fig. 1 und 2 dargestellten Anzeigesystemen 100 und 200 dadurch, dass es dazu ausgebildet ist, die Ausgabe von vier verschiedenen elektromedizinischen Geräten 105, 105', 105", 105'" anzuzeigen. Die vier verschiedenen elektromedizinischen Geräte 105, 105', 105", 105'" sind ausgebildet, ihre Daten an ein Kommunikationsnetzwerk 350 auszugeben. Dies erfolgt vorliegend durch eine jeweilige kabellose Verbindung, beispielsweise über WLAN, Bluetooth, BLE oder ZigBee. In dem dargestellten Ausführungsbeispiel umfasst das Kommunikationsnetzwerk sämtliche elektromedizinischen Geräte, die für die Behandlung eines einzigen Patienten genutzt werden. In einem alternativen nicht dargestellten Ausführungsbeispiel umfasst das Kommunikationsnetzwerk alle elektromedizinischen Geräte, die für die Messung eines bestimmten physiologischen Parameters oder Parametersatzes auf einer Krankenhausstation verwendet werden, um dadurch einen besonders guten Überblick über den entsprechenden physiologischen Zustand der Patienten dieser Station zu erhalten.

Die Anzeigesignaleinheit 320 ist hierbei dazu ausgebildet, das Gerätesignal 108, welches die Daten 107 umfasst, direkt von dem Kommunikationsnetzwerk 350 zu empfangen.

In dem dargestellten Ausführungsbeispiel ist das Anzeigesystem 300 genauso wie das Anzeigesystem 200 aus Fig. 2 aufgebaut, mit dem einzigen Unterschied, dass die Anzeigesignaleinheit 320 und die Anzeigesignalüberwachungseinheit 330 in einem gemeinsamen Gehäuse 360 angeordnet sind, welches von einem Anzeigegehäuse 365 der Anzeigeeinheit 110 beabstandet ausgebildet ist. Die sich dadurch ergebenden zwei Module des Anzeigesystems 300 verfügen über eine Kabel-basierte Verbindung miteinander, über die das Anzeigesignal 112 und das Ausgabesignal 118 übertragen werden. Das gemeinsame Gehäuse 360 verfügt über die an dem Gehäuse angeordnete Benutzerschnittstelle 242, bei der es sich vorliegend wiederum um eine Tastatur handelt. Weiterhin ist in dem gemeinsamen Gehäuse 360 ein optisches Ausgabeelement, insbesondere eine LED, angeordnet, die abhängig von der bestimmten Prüfinformation und mithin dem Prüfsignal eine optische Ausgabe 362 bereitstellt. Vorliegend leuchtet die LED nicht, falls die aktuelle visuelle Signatur mit der ausgegebenen visuellen Signatur übereinstimmt, und die LED leuchtet, vorzugsweise rot, falls die aktuelle visuelle Signatur nicht mit der ausgegebenen visuellen Signatur übereinstimmt.

Fig. 4 zeigt eine schematische Darstellung eines ersten Ausführungsbeispiels einer visuellen Ausgabe 114 des erfindungsgemäßen Anzeigesystems 300.

Die visuelle Ausgabe 114 verfügt über eine Vielzahl von gestrichelt dargestellten Ausgabebereichen 410, 410', 410". Die konkreten Daten-spezifischen Inhalte der Ausgabebereiche 410, 410', 410" wurden aus Gründen der Übersichtlichkeit nicht dargestellt. Die Darstellung von gestrichelten oder geschlossenen Rahmen, die im Folgenden verwendet werden, dient lediglich der besseren Visualisierung der Ausgabebereiche 410, 410', 410", wobei vorzugsweise für einen Nutzer des Anzeigesystems 300 keine Rahmen innerhalb der visuellen Ausgabe 114 erkennbar sind. Die Ausgabebereiche unterscheiden sich dabei in ihrer Größe. In dem dargestellten Ausführungsbeispiel hat die Anzeigeeinheit viermal Daten empfangen, die einem Visualisierungstypen zugeordnet wurden, dem ein hoher Relevanzlevel zugeordnet ist. Diese vier Ausgaben entsprechen den vier großen Ausgabebereichen 410. Ein Beispiel für einen solchen Visualisierungstyp mit hohem Relevanzlevel ist ein physiologischer Messwert, wie beispielsweise die Sauerstoffsättigung im Blut. Weiterhin wurden Daten mit einem mittleren Relevanzlevel empfangen, denen acht Ausgabebereiche 410' mittlerer Größe zugeordnet wurden. Letztlich wurden auch Daten mit einem geringen Relevanzlevel enthalten, denen sechs Ausgabebereiche 410" kleiner Größe durch die Anzeigesignaleinheit zugeordnet wurden.

Die großen Ausgabebereiche 410 sind sowohl im oberen Bereich, als auch im zentralen Bereich der visuellen Ausgabe 114 angeordnet und dadurch auch aufgrund ihrer Position für einen Nutzer des Anzeigesystems 300 schnell zu erkennen.

Die Anordnung der Ausgabebereiche und die Auswahl von deren Größe wurden von der Anzeigesignaleinheit aufgrund der aktuell darzustellenden Visualisierungstypen dynamisch bestimmt. Für den Fall, dass aktuell andere Visualisierungstypen darzustellen wären, würde mithin das gleiche Anzeigesystem eine andere Anordnung von Ausgabebereichen vorsehen.

Die Darstellungsgröße und die Darstellungsposition von empfangen Daten bilden in diesem Ausführungsbeispiel Darstellungseigenschaften. Die Gesamtheit aller Darstellungseigenschaften bildet die jeweilige Darstellungsart von empfangen Daten. Nicht dargestellt ist in Fig. 4, dass eine Schriftart und eine Schriftfarbe der darzustellenden Daten ebenfalls eine Darstellungseigenschaft bilden.

In einem nicht dargestellten erfindungsgemäßen Ausführungsbeispiel bilden lediglich die Darstellungsposition und eine Information, ob eine Darstellung der Daten erfolgt, eine jeweilige Darstellungseigenschaft der dynamisch bestimmten Darstellungsart.

Figs. 5 und 6 zeigen eine schematische Darstellung der visuellen Ausgabe 114 des erfindungsgemäßen Anzeigesystems für die Anzeige eines Alarmzustandes 420 (Fig. 5) und für die Anzeige einer nicht vollständigen Ausgabe 430 von empfangenen Daten (Fig. 6).

Die in Fig. 5 und 6 dargestellten visuellen Ausgaben 114 gehen von der in Fig. 4 gezeigten visuellen Ausgabe 114 aus, und zeigen, wie diese visuelle Ausgabe 114 durch die Anzeige eines Alarmzustand ist 420 oder durch die Anzeige einer nicht vollständigen Ausgabe 430 verändert wird.

Es ist zu erkennen, dass die Anzeige eines Alarmzustandes 420 zu einer Verschiebung und Verkleinerung von anderen Ausgabebereichen 410, 410', 410" führt. Die Anzeige eines Alarmzustandes 420 ist zentral auf der visuellen Ausgabe 114 dargestellt. Hierdurch kann ein Nutzer des Anzeigesystems besonders schnell das Vorliegen des Alarmzustandes erfassen.

Die Anzeige einer nicht vollständigen Ausgabe 430 erfolgt typischerweise in einem Randbereich der visuellen Ausgabe 114, vorliegend am oberen Rand. Die Anzeige einer nicht vollständigen Ausgabe 430 informiert einen Nutzer des Anzeigesystems darüber, dass aufgrund der Dimensionierung der visuellen Ausgabe nicht alle von dem Anzeigesystem empfangenen Daten durch die Anzeigeeinheit visuell ausgegeben werden können. Diese Anzeige führt ebenfalls dazu, dass die anderen Ausgabebereiche 410, 410', 410" verschoben und verkleinert werden.

Weiterhin ist in Fig. 5 und 6 ein besonders kleiner Ausgabebereich 410'" für die visuelle Signatur am unteren Rand der visuellen Ausgabe 114 vorgesehen. Die darin enthaltene visuelle Signatur ist für einen Nutzer des Anzeigesystems uninteressant und dient lediglich einer Überprüfung der Anzeigesignaleinheit durch die Anzeigesignalüberwachungseinheit, wie oben im Detail dargelegt. In einem nicht dargestellten Ausführungsbeispiel ist für die Signatur weiterhin eine Schriftfarbe gewählt, die ähnlich zu einer verwendeten Hintergrundfarbe ist, sodass die visuelle Signatur für einen Nutzer des Anzeigesystems nicht störend ist.

Die dargestellten Ausgabebereiche 410, 410', 410", 410'" sind nicht einschränkend zu verstehen. In nicht dargestellten Ausführungsbeispielen sind andere Größen, Positionen und Formen von Ausgabebereichen vorgesehen. Die Daten können dabei innerhalb der Ausgabenbereiche in Form von Buchstaben, Ziffern, Symbolen, Tabellen, Diagrammen und von jeglicher weiteren graphischen Darstellung ausgegeben werden.

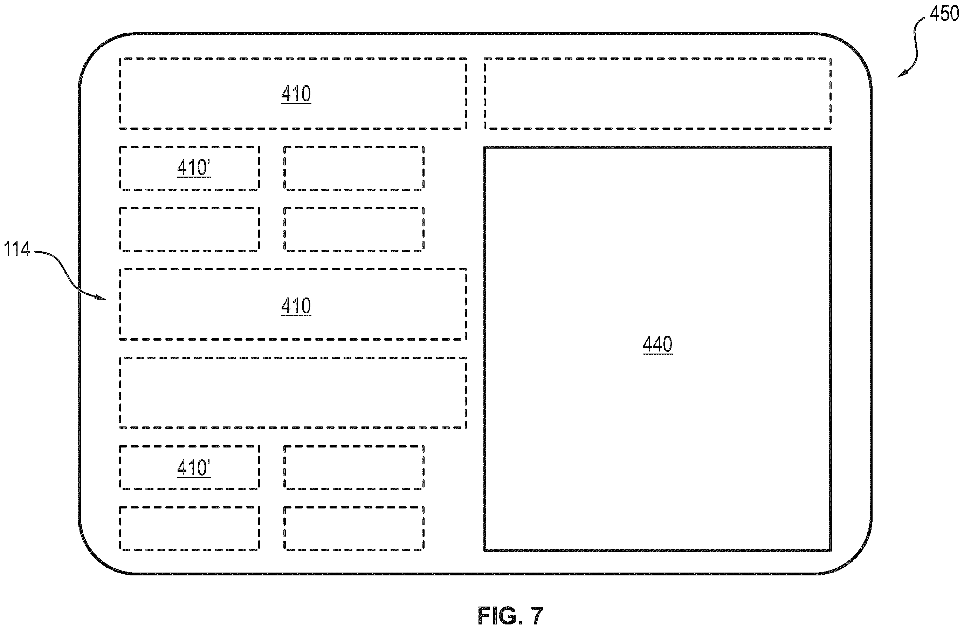

Fig. 7 zeigt eine schematische Darstellung der visuellen Ausgabe des erfindungsgemäßen Anzeigesystems für eine Überlappung der visuellen Ausgabe durch eine zusätzliche durch ein administratives Anzeigesignal ausgelöste Ausgabe 440.

In dem dargestellten Ausführungsbeispiel ist die Anzeigeeinheit weiterhin ausgebildet, ein zusätzliches administratives Anzeigesignal zu empfangen und basierend auf dem Anzeigesignal und dem zusätzlichen administrativen Anzeigesignal eine kombinierte visuelle Ausgabe 450 darzustellen, wobei das zusätzliche administrative Anzeigesignal nicht-medizinische Daten eines externen Gerätes indiziert. Die kombinierte Ausgabe 450 umfasst dabei die zusätzliche Ausgabe 440, die die ursprüngliche, in Fig. 4 dargestellte visuelle Ausgabe 114 überlagert, und einen Teil der ursprünglichen visuellen Ausgabe 114. Durch die Anzeigesignalüberwachungseinheit wurden die einzelnen Ausgabebereiche 410, 410', 410" über eine entsprechende Anpassung des Anzeigesignals angepasst. Diese Anpassung erfolgte derart, dass die vier großen Ausgabebereiche 410, denen ein hohes Relevanzlevel zugeordnet ist, weiterhin gut erkennbar und in ihrer Darstellungsposition nur leicht verändert sind. Weiterhin sind die acht Ausgabebereiche 410' mittlerer Größe, denen ein mittleres Relevanzlevel zugeordnet ist, derart ausgebildet, dass sie in ihrer Größe gegenüber der ursprünglichen, in Fig. 4 dargestellten Ausgabe 114, leicht reduziert und in ihrer Darstellungsposition verändert sind. Die in Fig. 4 dargestellten Ausgabebereiche kleiner Größe, denen lediglich ein geringes Relevanzlevel zugeordnet ist, wurden wegen der zusätzlichen Ausgabe 440 aus der kombinierten Ausgabe 450 entfernt.

Fig. 7 illustriert, wie eine kombinierte Ausgabe 450 die zugeordneten Relevanzlevel berücksichtigen kann, um trotz einer nicht beeinflussbaren Überlappung durch die zusätzliche Ausgabe 440 alle relevanten Daten in einer für einen Nutzer geeigneten Weise darzustellen.

In einem nicht dargestellten Ausführungsbeispiel wird neben der zusätzlichen Ausgabe auch eine visuelle Signatur durch die Anzeigeeinheit ausgegeben.

In einem weiteren nicht dargestellten Ausführungsbeispiel wird neben der zusätzlichen Ausgabe auch angezeigt, dass nicht alle empfangenen Daten ausgegeben werden konnten. Im dargestellten Fall wurde eine solche Anzeige einer nicht vollständigen Ausgabe nicht dargestellt, weil lediglich Daten, denen ein geringes Relevanzlevel zugeordnet ist, nicht dargestellt wurden. Hätten besonders relevanten Daten nicht dargestellt werden können, wäre eine entsprechende Anzeige einer nicht vollständigen Ausgabe erfolgt.

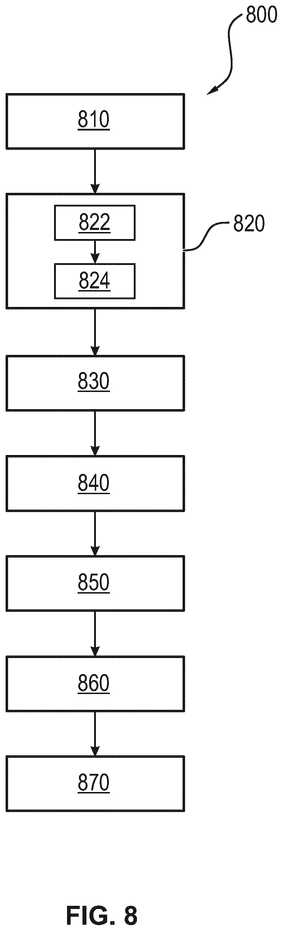

Fig. 8 zeigt ein Flussdiagramm eines ersten Ausführungsbeispiels eines erfindungsgemäßen Verfahrens 800.

Das erfindungsgemäße Verfahren 800 zum Anzeigen einer Ausgabe mindestens eines elektromedizinischen Gerätes umfasst eine Abfolge der im Folgenden beschriebenen Schritte in der dargestellten Reihenfolge.

Ein erster Schritt 810 umfasst ein Empfangen von Daten, die das mindestens eine elektromedizinische Gerät betreffen.

Ein darauffolgender Schritt 820 umfasst in einem ersten Teilschritt 822 ein Zuordnen der empfangenen Daten zu einem jeweiligen Visualisierungstyp und in einem zweiten Teilschritt 824 ein dynamisches Bestimmen einer jeweiligen Darstellungsart der empfangenen Daten basierend auf den aktuell darzustellenden Visualisierungstypen.

Ein weiterer Schritte 830 umfasst ein Erzeugen und Ausgeben eines Anzeigesignals basierend auf den empfangen Daten und der jeweils bestimmten Darstellungsart, wobei die bestimmte Darstellungsart von Daten aus einer vorbestimmten Teilmenge von besonders relevanten Datenarten zumindest als Darstellungseigenschaft umfasst, dass eine Darstellung als für einen Nutzer erkennbare visuelle Ausgabe erfolgt, und wobei die bestimmte Darstellungsart weiterhin als Darstellungseigenschaft eine Darstellungsposition umfasst.

Ein nächster Schritt 840 umfasst ein Empfangen des Anzeigesignals und ein Darstellen einer visuellen Ausgabe basierend auf dem Anzeigesignal.

Ein weiterer Schritt 850 umfasst ein Senden eines Ausgabesignals, das Informationen zur erfolgten visuellen Ausgabe enthält.

Ein darauffolgender Schritt 860 umfasst ein Empfangen des Anzeigesignals und des Ausgabesignals und ein Vergleichen der beiden Signale miteinander.

Ein letzter Schritt 870 umfasst ein Ausgeben eines Warnsignals, falls eine mangelnde Übereinstimmung zwischen erfolgter visueller Ausgabe und gemäß Anzeigesignal zu erfolgender visueller Ausgabe vorliegt.

Die Schritte 810 bis 860 werden hintereinander ausgeführt. Dies erfolgt vorzugsweise in regelmäßigen zeitlichen Abständen, die so gewählt sind, dass die visuelle Ausgabe nahezu in Echtzeit mit dem Empfang der Daten erfolgt. Der Schritt 870 wird dabei nicht regelmäßig ausgeführt, da er lediglich im Fehlerfall, nämlich bei einer mangelnden Übereinstimmung zwischen erfolgter visueller Ausgabe und zu erfolgender visueller Ausgabe, ausgeführt wird.

Um einen aktuellen Zustand des elektromedizinischen Gerätes darstellen zu können, sind in einem nicht dargestellten Ausführungsbeispiel die regelmäßigen zeitlichen Abständen zwischen der jeweiligen Ausgabe des Anzeigesignals an die Anzeigeeinheit größer als 1 Sekunde, vorzugsweise größer als 2 Sekunden, besonders bevorzugt etwa 3 Sekunden lang. Hierdurch kann sichergestellt werden, dass nicht für jedes empfangene Signal ein einzelnes Anzeigesignal erzeugt wird, sondern dass eine Gruppe von physiologisch zusammengehörenden Signalen im Rahmen eines einzigen Anzeigesignals verarbeitet wird.

In einem anderen nicht dargestellten Ausführungsbeispiel löst das Empfangen von Daten direkt das dargestellte Verfahren 800 aus. Hierdurch kann nahezu in Echtzeit eine visuelle Ausgabe der empfangenen Daten erfolgen.

### Bezugszeichenliste

- 100, 200, 300: Anzeigesystem
- 105, 105', 105", 105"': elektromedizinisches Gerät
- 107: Daten
- 108: Gerätesignal
- 110: Anzeigeeinheit
- 112: Anzeigesignal
- 114: visuelle Ausgabe
- 116: Display
- 118: Ausgabesignal
- 120, 220, 320: Anzeigesignaleinheit
- 122: Kommunikationsschnittstelle
- 124: erster Verarbeitungsschritt
- 125: zweiter Verarbeitungsschritt
- 126: dritter Verarbeitungsschritt
- 130, 230, 330: Anzeigesignalüberwachungseinheit
- 132: Überwachungsschritt
- 134: Warnsignal
- 236: Signatursignal
- 238: Prüfsignal
- 240: Eingabeeinheit
- 241: Gesamtgehäuse
- 242: Benutzerschnittstelle
- 244: Eingabesignal
- 350: Kommunikationsnetzwerk
- 360: gemeinsames Gehäuse
- 362: optische Ausgabe
- 365: Anzeigegehäuse
- 410, 410', 410", 410'": Ausgabebereiche
- 420: Anzeige eines Alarmzustandes
- 430: Anzeige einer nicht vollständigen Ausgabe
- 440: zusätzliche Ausgabe
- 450: kombinierte Ausgabe
- 800: Verfahren
- 810, 820, 830, 840, 850, 860, 870: Verfahrensschritte
- 822, 824: Teilschritte des Verfahrensschrittes 720

## Patentansprüche

1. Anzeigesystem (100) zum Anzeigen einer Ausgabe eines elektromedizinischen Gerätes (105), mit
- einer Anzeigeeinheit (110), die ausgebildet ist, ein Anzeigesignal (112) zu empfangen und basierend auf dem Anzeigesignal (112) eine visuelle Ausgabe (114) darzustellen,
- einer Anzeigesignaleinheit (120), die eine Kommunikationsschnittstelle (122) zur Kommunikation mit dem elektromedizinischen Gerät (105) aufweist und ausgebildet ist, Daten (107), die das elektromedizinische Gerät (105) betreffen, über die Kommunikationsschnittstelle (122) zu empfangen, diese Daten (107) einem Visualisierungstyp zuzuordnen, abhängig von den aktuell darzustellenden Visualisierungstypen eine jeweilige Darstellungsart der empfangenen Daten (107) dynamisch zu bestimmen und basierend auf den Daten (107) und der jeweils bestimmten Darstellungsart das Anzeigesignal (112) zu erzeugen und an die Anzeigeeinheit (110) auszugeben, wobei die bestimmte Darstellungsart von Daten (107) aus einer vorbestimmten Teilmenge von besonders relevanten Datenarten zumindest als Darstellungseigenschaft umfasst, dass eine Darstellung als für einen Nutzer erkennbare visuelle Ausgabe (114) erfolgt, und wobei die bestimmte Darstellungsart weiterhin als Darstellungseigenschaft eine Darstellungsposition umfasst, und
- eine Anzeigesignalüberwachungseinheit (130), die mit der Anzeigeeinheit (110) und der Anzeigesignaleinheit (120) signaltechnisch verbunden ist, und die ausgebildet ist, das Anzeigesignal (112) zu empfangen und ein Ausgabesignal (118) von der Anzeigeeinheit (110) zu empfangen, wobei das Ausgabesignal (118) Informationen zur erfolgten visuelle Ausgabe (114) enthält, und wobei die Anzeigesignalüberwachungseinheit (130) ausgebildet ist, Anzeigesignal (112) und Ausgabesignal (118) miteinander zu vergleichen und im Falle einer mangelnden Übereinstimmung zwischen erfolgter visueller Ausgabe (114) und gemäß Anzeigesignal (112) zu erfolgender visueller Ausgabe ein Warnsignal (134) auszugeben.

2. Anzeigesystem (200) gemäß Anspruch 1, wobei die Anzeigesignalüberwachungseinheit (230) ausgebildet ist, das Warnsignal (134) an die Anzeigesignaleinheit (220) auszugeben.

3. Anzeigesystem (100) gemäß mindestens einem der vorhergehenden Ansprüche, wobei die vorbestimmte Teilmenge von besonders relevanten Datenarten zumindest Alarminformationen und/oder klinische Messwerte umfasst.

4. Anzeigesystem (100) gemäß mindestens einem der vorhergehenden Ansprüche, wobei die Anzeigesignaleinheit (120) weiterhin ausgebildet ist, das Anzeigesignal (112) derart zu erzeugen, dass ein entsprechender Hinweis (430) über die visuelle Ausgabe (114) dargestellt wird, falls aufgrund der Dimensionierung der visuellen Ausgabe (114) nicht alle von dem elektromedizinischen Gerät (105) empfangenen Daten durch die Anzeigeeinheit (110) visuell ausgegeben werden können.

5. Anzeigesystem (100) gemäß mindestens einem der vorhergehenden Ansprüche, wobei die Anzeigesignaleinheit (120) weiterhin ausgebildet ist, eine Darstellungsgröße und/oder die Darstellungsposition von empfangenen Daten (107) eines Visualisierungstyps abhängig von einem diesem Visualisierungstyp zugeordneten Relevanzlevel zu bestimmen.

6. Anzeigesystem (200) gemäß mindestens einem der vorhergehenden Ansprüche, wobei das Anzeigesystem weiterhin eine Eingabeeinheit (240) aufweist, die mit der Anzeigesignaleinheit (220) signaltechnisch verbunden ist und eine Benutzerschnittstelle (242) aufweist, wobei die Eingabeeinheit (240) ausgebildet ist, über die Benutzerschnittstelle (242) abhängig von einer Benutzereingabe eines Nutzers des Anzeigesystems (200) ein Eingabesignal (244) ausgeben, und
wobei die Anzeigesignaleinheit (220) weiterhin ausgebildet ist, das Eingabesignal (244) zu empfangen und abhängig von dem dem Eingabesignal (244) zugeordneten Visualisierungstyp eine entsprechende Darstellungsart zu bestimmen und ein Anzeigesignal (112) basierend auf allen aktuell empfangenen Daten (107) und der jeweils bestimmten Darstellungsart zu erzeugen und auszugeben.

7. Anzeigesystem (200) gemäß mindestens einem der vorhergehenden Ansprüche, wobei die Anzeigesignalüberwachungseinheit (230) weiterhin ausgebildet ist, eine sich in zeitlichen Abständen ändernde visuelle Signatur über ein Signatursignal (236) an die Anzeigesignaleinheit (220) auszugeben,
wobei die Anzeigesignaleinheit (220) weiterhin ausgebildet ist, die aktuelle visuelle Signatur über das Anzeigesignal (112) auszugeben, und
und wobei die Anzeigesignalüberwachungseinheit (230) weiterhin ausgebildet ist, über das Ausgabesignal (118) die aktuell dargestellte visuelle Signatur mit der aktuellen visuellen Signatur zu vergleichen und abhängig von diesem Vergleich eine Prüfinformation bereitzustellen.

8. Anzeigesystem (200) gemäß mindestens einem der vorhergehenden Ansprüche, wobei die Anzeigeeinheit weiterhin ausgebildet ist, ein zusätzliches administratives Anzeigesignal zu empfangen und basierend auf dem Anzeigesignal und dem zusätzlichen administrativen Anzeigesignal eine kombinierte visuelle Ausgabe (450) darzustellen, wobei das zusätzliche administrative Anzeigesignal nicht-medizinische Daten eines externen Gerätes indiziert.

9. Anzeigesystem (300) gemäß mindestens einem der vorhergehenden Ansprüche, wobei die Kommunikationsschnittstelle (122) ausgebildet ist, Daten von einer Mehrzahl von elektromedizinischen Geräten (105, 105', 105", 105"') zu empfangen, wobei der den Daten durch die Anzeigesignaleinheit zugeordnete Visualisierungstyp eine Geräteinformation umfasst, die das zu den Daten (107) korrespondierende elektromedizinische Gerät (105, 105', 105", 105'") indiziert.

10. Verfahren (800) zum Anzeigen einer Ausgabe eines elektromedizinischen Gerätes (105), aufweisend die folgenden Schritte
- Empfangen von Daten (107), die das elektromedizinische Gerät (105) betreffen;
- Zuordnen der empfangenen Daten (107) zu einem jeweiligen Visualisierungstyp und dynamisches Bestimmen einer jeweiligen Darstellungsart der empfangenen Daten (107) basierend auf den aktuell darzustellenden Visualisierungstypen;
- Erzeugen und Ausgeben eines Anzeigesignals (112) basierend auf den empfangen Daten (107) und der jeweils bestimmten Darstellungsart, wobei die bestimmte Darstellungsart von Daten (107) aus einer vorbestimmten Teilmenge von besonders relevanten Datenarten zumindest als Darstellungseigenschaft umfasst, dass eine Darstellung als für einen Nutzer erkennbare visuelle Ausgabe (114) erfolgt, und wobei die bestimmte Darstellungsart weiterhin als Darstellungseigenschaft eine Darstellungsposition umfasst;
- Empfangen des Anzeigesignals (112) und Darstellen einer visuellen Ausgabe (114) basierend auf dem Anzeigesignal (112);
- Senden eines Ausgabesignals (118), das Informationen zur erfolgten visuellen Ausgabe (114) enthält;
- Empfangen des Anzeigesignals (112) und des Ausgabesignals (118) und Vergleichen der beiden Signale miteinander;
- Ausgeben eines Warnsignals (134), falls eine mangelnde Übereinstimmung zwischen erfolgter visueller Ausgabe (114) und gemäß Anzeigesignal (112) zu erfolgender visueller Ausgabe vorliegt.

11. Verfahren gemäß Anspruch 10, wobei das Erzeugen des Anzeigesignals (112) derart erfolgt, dass ein entsprechender Hinweis (430) über die visuelle Ausgabe (114) dargestellt wird, falls aufgrund der Dimensionierung der visuelle Ausgabe (114) nicht alle von dem elektromedizinischen Gerät (105) empfangenen Daten (107) visuell ausgegeben werden können.

12. Verfahren nach einem der Ansprüche 10 oder 11, weiterhin aufweisend die Schritte
- Ausgeben von einer sich in zeitlichen Abständen ändernden visuellen Signatur über ein Signatursignal (236);
- Empfangen des Signatursignals (236) und Ausgeben der aktuellen visuellen Signatur über das Anzeigesignal (112);
- Vergleichen der aktuell dargestellten visuellen Signatur aus dem Ausgabesignal (118) mit der aktuellen visuellen Signatur und Bereitstellen einer Prüfinformation abhängig von diesem Vergleich.

13. Verfahren nach mindestens einem der Ansprüche 10 bis 12, wobei das Verfahren zum Anzeigen einer Ausgabe einer Mehrzahl von elektromedizinischen Geräten (105, 105', 105", 105"') ausgestaltet ist, mit dem zusätzlichen Schritt,
- Empfangen von Daten (107), die mindestens ein weiteres elektromedizinisches Gerät (105', 105", 105'") betreffen, wobei der den Daten (107) zugeordnete Visualisierungstyp eine Geräteinformation umfasst, die das zu den Daten (107) korrespondierende elektromedizinische Gerät (105, 105', 105", 105'") indiziert.

14. Programm mit einem Programmcode zur Durchführung des Verfahrens (800) gemäß mindestens einem der Ansprüche 10 bis 13, wenn der Programmcode auf einem Computer, einem Prozessor oder einer programmierbaren Hardwarekomponente ausgeführt wird.
